# EUROPEAN PATENT SPECIFICATION

(11) **EP 4 331 548 B1**
(45) Date of publication and mention of the grant of the patent: **09.10.2024**
(21) Application number: 23219567.7
(22) Date of filing: 05.03.2021
(51) Int. Cl.: A61F 13/49

(54) **ABSORBENT ARTICLE WITH IMPROVED ELASTIC PANELS**
ABSORBIERENDER ARTIKEL MIT VERBESSERTEN ELASTISCHEN ELEMENTEN
ARTICLE ABSORBANT AVEC PANNEAUX ÉLASTIQUES AMÉLIORÉS

(43) Date of publication of application: 06.03.2024
(62) Divisional of application: 21161108.2
(73) Proprietor: Ontex BV, 9255 Buggenhout (BE); Ontex Group NV, 9320 Erembodegem (BE)
(72) Inventor: LAMPE, Ulrich, 56727 Mayen (DE); GÖTZ, Sven, 53119 Bonn (DE); COBBAERT, Dries, 1770 Liedekerke (BE)
(74) Representative: Macchetta, Andrea

(56) References cited:
- EP-A1- 1 803 428
- EP-A1- 3 213 728
- WO-A1-2020/164378

## Description

### TECHNICAL FIELD

The disclosure relates to absorbent articles such as disposable absorbent articles, preferably selected from the group consisting of diapers (whether for baby or adults), pants (whether for baby or adults), briefs, and combinations thereof.

### BACKGROUND

Disposable absorbent articles, such as diapers, are designed to contain bodily wastes and prevent soiling of the wearer's clothing and/or other items (e.g., a bed, a chair, a blanket, etc.). The fit of the article to the wearer's body is important in ensuring that these wastes are contained. Such articles are also designed to be cost-effective, and therefore manufacturers generally make the articles applicable for use by individuals with a wide range of body types. Accordingly, new and improved disposable absorbent articles that both conform to a wide range of body types and fit snuggly to the user to contain wastes and limit leakage are of continued interest.

One way in which manufacturers attempt to balance the competing interests of proper fit and variation in body type is through the use of expandable materials. One such group of materials is known as stretch laminates. As the name suggests, these materials are actually composites of individual components that are laminated together, through the use of an adhesive, for example. A typical stretch laminate will attempt to combine one or more layers of cover material with one or more layers or strands of an elastomeric material.

Complications arise in that stretch laminates are notoriously difficult and expensive to manufacture. Considerable effort has gone into proposing new types of stretch laminates and new methods for the fabrication of stretch laminates.

For example, EP 3 213 728 A1 relates to transversely extensible elastic laminar web materials comprising - a first and a second web material each of which defines a first and a second distal region adjacent to corresponding longitudinal side edges and a central region between the aforesaid distal regions - at least one web of elastomeric material applied to these central regions of the first and second web materials and - a plurality of connection formations applied to at least one distal region of said first and second web materials, and projecting from a respective longitudinal edge. In the transversely extensible elastic laminar web material, the elastomeric web material and the connection formations are interposed between said first and second web materials and are joined thereto by mechanical welds. EP 3 496 692 A1 relates to an absorbent article that includes a first waist region, a second waist region and a crotch region disposed between the first and second waist regions; and a chassis comprising a topsheet, a backsheet and an absorbent core disposed between the topsheet and the backsheet. The absorbent article also includes an ear disposed in one of the waist regions. The ear includes a laminate having a first nonwoven and a second nonwoven and an elastomeric material sandwiched between said first and second nonwovens in an elasticized region. The laminate also includes a first bonding region comprising a first plurality of ultrasonic bonds having a first bond density, and a second bonding region comprising a second plurality of ultrasonic bonds having a second bond density. The first bond density is greater than the second bond density. Other relevant prior art are EP 1803428 and WO 2020/164378.

There is however a continuing need to provide new stretch laminates, that are better performing and/or cheaper to manufacture, and new absorbent articles that comprise such stretch laminates. In particular, it is highly desirable that such new stretch laminates provide both an increase in actual and perceived softness.

### SUMMARY

The invention relates to the article according to claim 1.

### BRIEF DESCRIPTION OF THE FIGURES

**Fig. 1** Illustrates an absorbent article according to an embodiment of the present disclosure.
Fig. 2A-B Illustrates absorbent articles according to an embodiment of the present disclosure.
**Fig. 3** Illustrates a cross-section at a position A-A of Fig. 2A.
**Fig. 4** Illustrates an elastically elongatable panel according to an embodiment of the present disclosure.
**Fig. 5** Illustrates a schematic illustration of a bonding pattern for an elastically elongatable panel according to an embodiment of the present disclosure.
**Fig. 6** Illustrates a schematic illustration of wrinkles at an exemplary location of a sample elastically elongatable panel according to the wrinkle distribution test method herein.
**Fig. 7** Illustrates an elastically elongatable panel according to an embodiment of the present disclosure.
**Fig. 8** Illustrates an elastically elongatable panel according to an embodiment of the present disclosure.
**Fig. 9** Illustrates discrete bonding elements according to an embodiment of the disclosure.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" (weight percent), here and throughout the description unless otherwise defined, refers to the relative weight of the respective component based on the overall weight of the formulation.

The use of the term "layer" can refer, but is not limited, to any type of substrate, such as a woven web, nonwoven web, films, laminates, composites, elastomeric materials, absorbent materials (such as SAP and cellulose fibers/fluff mixtures), or the like. A layer can be liquid and air permeable, permeable to air but impermeable to liquids, impermeable both to air and liquid, or the like. When used in the singular, it can have the dual meaning of a single element or a plurality of elements, such as a laminate or stacked plural sub-layers forming a common layer.

"Laminate" refers to elements being attached together in a layered arrangement.

By "substantially", it is meant at least the majority of the structure referred to.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

The term "absorbent article" refers to a device that absorbs and contains liquid, and more specifically, refers to a device that is placed against or in proximity to the body of the wearer to absorb and contain the various wastes/exudates discharged from the body.

The terms "activated" and "pre-activated" refer to a process of mechanically deforming a material in order to increase the extensibility of at least a portion of the material. A material may be activated or pre-activated by, for example, incrementally stretching the material in at least one direction.

The terms "adhesively bonded" or "adhesively laminated" refer to a laminate wherein an adhesive is used to bond an elastomeric material to at least one cover layer.

The term "attached" refers to elements being connected or united by fastening, adhering, bonding, or by any other method suitable for connecting the elements together and to their constituent materials. Many suitable methods for attaching elements together are well-known, including adhesive bonding, pressure bonding, thermal bonding, ultrasonic bonding, mechanical fastening, etc. Such attachment methods may be used to attach elements together over a particular area either continuously or intermittently.

The term "diaper" refers to an absorbent article generally worn by infants or incontinent persons about the lower torso and having the general form of a sheet, different portions of which are fastened together to encircle the waist and the legs of the wearer.

The term "disposable" refers to absorbent articles that generally are not intended to be laundered or otherwise restored or reused as absorbent articles, i.e., they are intended to be discarded after a single use and, preferably, to be recycled, composted or otherwise disposed of in an environmentally compatible manner.

The term "disposed" is used to mean that an element(s) is formed (joined and positioned) in a particular place or position as a unitary structure with other elements or as a separate element joined to another element.

The term "extensible" refers to the property of a material, wherein: when a biasing force is applied to the material, the material can be extended to an elongated length of at least 110% of its original relaxed length (i.e., can extend 10%), without a rupture or breakage that renders the material unusable for its intended purpose. A material that does not meet this definition is considered inextensible. In some embodiments, an extensible material may be able to be extended to an elongated length of 125% or more of its original relaxed length without rupture or breakage that renders the material unusable for its intended purpose. An extensible material may or may not exhibit recovery after application of a biasing force. Throughout the present disclosure, an extensible material is considered to be "elastically extensible" if, when a biasing force is applied to the material, the material can be extended to an elongated length of at least 110% of its original relaxed length (i.e., can extend 10%), without rupture or breakage which renders the material unusable for its intended purpose, and when the force is removed from the material, the material recovers at least 40% of its elongation. In various examples, when the force is removed from an elastically extensible material, the material may recover at least 60%, or at least 80%, of its elongation.

The terms "interior" and "exterior" refer respectively to the location of an element that is intended to be placed against or toward the body of a wearer when an absorbent article is worn and the location of an element that is intended to be placed against or toward any clothing that is worn over the absorbent article. Synonyms for "interior" and "exterior" include, respectively, "inner" and "outer", as well as "inside" and "outside", or "body-facing" and "garment-facing". Also, when the absorbent article is oriented such that its interior faces upward, e.g., when it is laid out in preparation for setting the wearer on top of it, synonyms include "upper" and "lower" and "top" and "bottom", respectively.

The term "joined" refers to configurations whereby an element is directly secured to another element by attaching the element directly to the other element, and configurations whereby an element is indirectly secured to another element by attaching the element to intermediate member(s) which in turn are attached to the other element.

The term "lateral" or "transverse" refers to a direction running at a 90 degree angle to the longitudinal direction and when combined with the term "substantially" includes directions within ±45° of the lateral direction.

The term "longitudinal" refers to a direction running parallel to the maximum linear dimension of the article and when combined with the term "substantially" includes directions within ±45° of the longitudinal direction.

The term "pant" or "pants" refers to an absorbent article generally worn by infants and incontinent persons (whether infants or adults) about the lower torso and that can be applied or removed from the wearer without unfastening. A pant typically comprises a front and back elastic belt portions (or elastic panels) and a crotch portion connecting said belt portions, the belts are typically joined together at lateral seams so as to provide a waste opening circumscribed by the belts and two leg openings circumscribed by the belts and/or crotch portion. The pant may be placed in position on the wearer by inserting the wearer's legs into the leg openings and sliding the pant into position about the wearer's lower torso. While the term "pant" is used herein, pants are also commonly referred to as "closed diapers", "prefastened diapers", "pull-on diapers", "training pants" and "diaper-pants".

The term "recovery" refers to ability of a material to return to its original size after it has been stretched.

The term "refastenable" refers to the property of two elements being capable of releasable attachment, separation, and subsequent releasable reattachment without substantial permanent deformation or rupture.

The terms "releasably attached," "releasably engaged," and variations thereof refer to two elements being connected or connectable such that the elements tend to remain connected absent a separation force applied to one or both of the elements, and the elements being capable of separation without substantial permanent deformation or rupture. The required separation force is typically beyond that encountered while wearing the absorbent garment.

The term "wrinkle" as used herein refers to a fold, ridge or crease.

Embodiments of the articles and processes according to the disclosure will now be described. It is understood that technical features described in one or more embodiments maybe combined with one or more other embodiments without departing from the intention of the disclosure and without generalization therefrom, especially when such combinations are explicitly or implicitly inferred.

### THE ABSORBENT ARTICLE

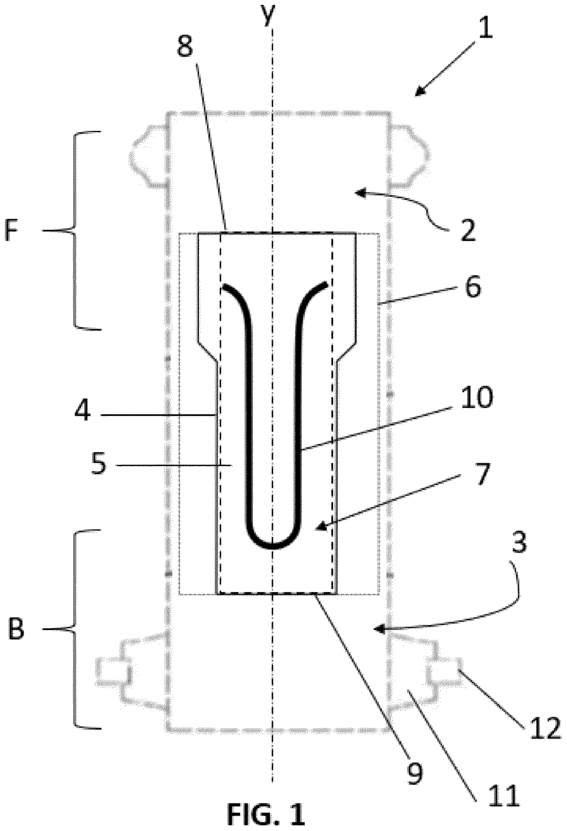

As exemplified in Fig. 1, absorbent articles herein comprise a chassis comprising a liquid permeable topsheet (2), a liquid impermeable backsheet (3), and an absorbent core (4) positioned between said topsheet (2) and backsheet (3), and comprising a longitudinal axis (y) extending along a length of said chassis and a transverse axis being substantially perpendicular to the longitudinal axis and extending along a width of said chassis, wherein said length extends along the longest dimension of said chassis. Articles herein are preferably selected from diapers or pants (whether refastenable or not).

The absorbent core (4) comprises an absorbent material (5), said absorbent material comprising cellulose fibers and/or superabsorbent polymers, preferably wherein said absorbent material is contained within at least one core wrap substrate (6) enclosing said absorbent material therein, typically wherein the superabsorbent polymers are in the form of particles or granules, fibers, and mixtures thereof. The core wrap is typically a nonwoven selected from the group consisting of spunbond (S), spunbond-meltblown (SM), spunbond-metlblown-spunbond (SMS), spunbond-meltblown-metlblown-spunbond (SMMS), spunbond-spunbond-metlblown-spunbond (SSMS), spunbond-spunbond-metlblown-spunbond-spunbond (SSMSS), and combinations thereof. The core wrap may also, or alternatively, comprise a carded nonwoven preferably a carded thermobonded nonwoven wherein the thermobonds are formed by calendering (i.e. a carded nonwoven free of air-through bonding).

The absorbent article may further comprise an acquisition distribution layer (ADL) (7) positioned between the topsheet (2) and an upper layer of the core wrap (5). The ADL may be in good direct contact with both the topsheet (2) and an upper layer of the core wrap (5) over the majority of its surface area. ADLs customary in the art may be used such as nonwovens selected from spunbond or carded thermobonded whether air-through bonded or calendered. Also ADLs comprising cellulosic fibers and/or polylactide are suitable.

The absorbent article typically comprises a front region (F), a back region (B), and a crotch region positioned between the front region (F) and back region (B) such that the longitudinal axis (y) crosses each of said front region (F), crotch region and back region (B) generally in this order. The crotch region of the article typically being positioned to fit between the wearer's legs when the article is worn by a subject.

The absorbent core (4) of the article (1) may comprise a front transversal edge (8) generally positioned at or proximal to the front region (F) and a back transversal edge (9) generally positioned at or proximal to the back region (B), and typically connected to each other by oppositely disposed lateral edges extending along the longitudinal axis (y). The transversal edges (8, 9) and lateral edges together forming the perimeter of said core (4)

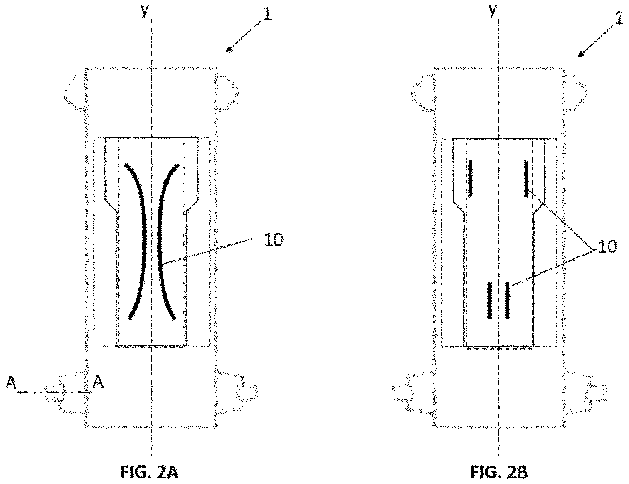

The absorbent core may further comprise one or more channels (10) substantially free of absorbent material, typically meaning less than 15%wt or less than 5% of absorbent material within said channels. Preferably, a top layer of the core wrap (5) is bonded to a bottom layer of the core wrap (5) in one or more attachment zones corresponding to said channels (10) thereby allowing the channels to be substantially free of absorbent material or even essentially entirely free of absorbent material. The bonding may be achieved via adhesive and/or mechanical bonding. Although Fig. 1 illustrates a single said channel (10) other channel shapes and/or plural channels are also contemplated as exemplified, without any limitation intended, in Figs. 2A-B.

The absorbent articles herein comprise at least one elastically elongatable panel (11) joined to the chassis as will be explained more in detail in the section that follows. Said panel(s) are typically laterally extending in the back portion (B) of the article (1) when the article is a diaper, whilst when the article is a pant may be rather front and/or back panels forming front and/or back belts at the front portions (F) and/or back portion (B).

The elastically elongatable panel (11) may each comprise a fastening member (12) arranged to fasten to a frontal area of the external surface of the article. Typically the fastening member (12) comprises adhesive and/or mechanical hooks that mate and/or couple to a nonwoven layer such as a landing zone positioned at the external surface of the article at a belly position when worn by a subject.

In an embodiment, the at least one elastically elongatable panel (11) is joined to the chassis by mechanical bonding or adhesive at a first attachment zone of said panel, and preferably wherein said panel (11) is joined, preferably by mechanical bonding or adhesive, to a fastening member (12) at a second attachment zone of said panel oppositely disposed from said first attachment zone at an opposite end of said panel.

Absorbent articles herein may further comprise additional components such as lateral and/or transversal cuffs arranged to provide liquid leakage barriers at the longitudinal and/or transversal edges of the article respectively; frontal (typically inelastic) panels; one or more skin care lotions on the topsheet; one or more odor control compositions and/or fragrances contained in one or more layers beneath the topsheet; and other components common in the art of diapers and pants.

### ELASTIC PANEL(S)

Elastically elongatable panel(s) (11) herein are joined to the chassis, and comprise: at least one, preferably at least two, cover layers (13, 13'); and an elastomeric film (14) attached to the cover layer, wherein said least one cover layer (13, 13') and elastomeric film (14) are joined by mechanical bonding, at a plurality of discrete bonding elements (15), preferably having a shape selected from substantially square, rectangular, circular and combinations thereof, and wherein each said panel (11) comprises a plurality of wrinkles having peaks (16) and troughs (17) formed on at least one, preferably both, of said cover layer (13, 13'), wherein said panel (11) comprises an average of no more than two, preferably no more than one, wrinkles between two consecutive discrete bonding elements (15) along a panel length (L) extending substantially parallel to the transverse axis, and wherein said panel (11) comprises a wrinkle distribution of at least 1.1, preferably from 1.2 to 2.5, even more preferably from 1.3 to 2, wrinkles/mm, according to the method herein. Advantageously, a large number of wrinkles within the above ranges generally increases bulkiness and therefore actual and perceived softness of the material which is ever more important in elastic side panels which are in tight contact with the skin of the user, nevertheless if the number of wrinkles is too high bulkiness turns into roughness as the peaks tend to form sharper apexes at extremely short amplitudes therefore it is beneficial to even more preferably remain within the above cited preferred range.

In an alternative aspect of the disclosure, the elastically elongatable panel(s) (11) herein are joined to the chassis, and comprise: at least one, preferably at least two, cover layers (13, 13'); and a plurality of elastic strands (14') attached to the cover layer, wherein said least one cover layer (13, 13') and plurality of elastic strands (14') are joined by mechanical bonding, at a plurality of discrete bonding elements (15), preferably having a shape selected from substantially square, rectangular, circular and combinations thereof, and wherein each said panel (11) comprises a plurality of wrinkles having peaks (16) and troughs (17) formed on at least one, preferably both, of said cover layer (13, 13'), wherein said panel (11) comprises an average of no more than two, preferably no more than one, wrinkles between two consecutive discrete bonding elements (15) along a panel length (L) extending substantially parallel to the transverse axis, and wherein said panel (11) comprises a wrinkle distribution of at least 1.1, preferably from 1.2 to 2.5, even more preferably from 1.3 to 2, wrinkles/mm, according to the method herein. The same advantages reported in the above paragraph are observed and moreover allows for a further reduction in cost as well as an improvement in breathability thanks to the voids present between elastic strands. It is understood by a person skilled in the art, that all embodiments herein referring to a film (14) may further apply and be combined to this aspect comprising elastic strands (14').

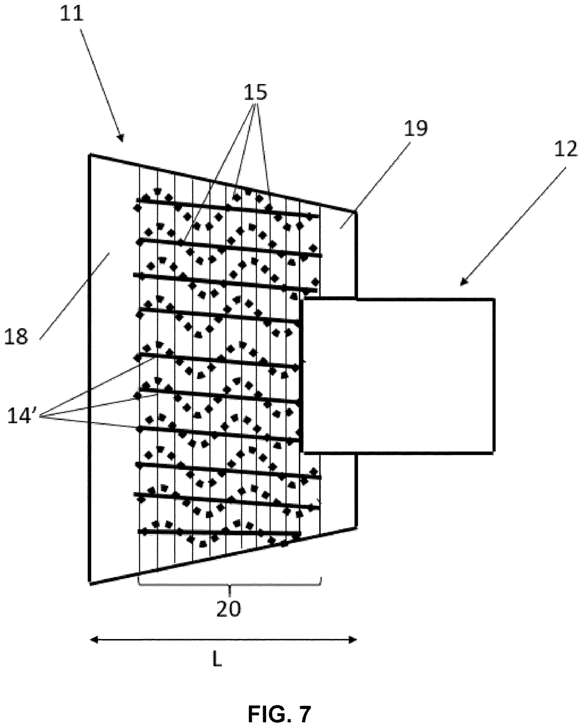

In an embodiment, as exemplified in Fig. 7, the plurality of elastic strands (14') are spaced apart from each other along an axis substantially perpendicular to the panel length (L), typically such that voids are formed between each consecutive elastic strands (14') that are free of elastic material. Preferably wherein the spacing between consecutive elastic strands (14') is greater than 0.03L, preferably from 0.035L to 0.35L, preferably from 0.05L to 0.30L, more preferably from 0.08L to 0.25L, even more preferably from 0.09L to 0.20L, even more preferably from 0.10L to 0.15L, where L refers to the panel length (L). Advantageously this allows for further breathability which reduces sweating that is normally observed when a film is used (even if the film comprises micro-apertures to allow some vapor permeability). Preferably, by staying within the specified upper limits a more evenly distributed elastic behavior is observed, in addition to the excellent breathability, that further allows to limit risks of necking of the laminate upon stretching.

In a highly preferred embodiment, and especially preferable when a plurality of elastic strands (14') are used, the plurality of discrete bonding elements (15) are substantially sinusoidally disposed (as will be explained in the below paragraphs and when reference is made to a film (14) such still applies to elastic strands (14') herein). Preferably, the substantially sinusoidally disposed discrete bonding elements (15) (herein also referred to as "sinusoidally oriented discrete bonding elements (15)") are aligned such that when an imaginary line is traced between bonding elements substantially sinusoidal waves are formed along the panel length (L), and wherein each of the plurality of elastic strands (14') crosses said imaginary line at a plurality of intersections, preferably at least 4 intersections, even more preferably at least 5 intersections, even more preferably from 6 to 30 intersections, even more preferably from 7 to 25 intersections, even more preferably from 8 to 20 intersections, even more preferably from 9 to 15 intersections, as exemplary illustrated in Fig. 7. Advantageously this allows for anchoring the elastic strands whilst achieving superior softness compared to for example strand coating with adhesive as customary in the art and or full coating of adhesive. Moreover, compared to mechanically bonding in alternate ways e.g. with linear bonds it allows to achieve better anchoring whilst allowing some free-movement of the elastic strands between intersections thus contributing not only to softness but further mechanical performance in an effective and cost-effective manner.

A further advantage of using sinusoidally disposed discrete bonding elements (15) as described herein is that the bonding pattern density may be kept substantially the same thus saving on process complexity and limiting the risk of unsightly wrinkle variation. It is therefore preferred, though not essential, herein that when sinusoidally disposed discrete bonding elements are used, the bond density throughout the panels (11) is substantially the same.

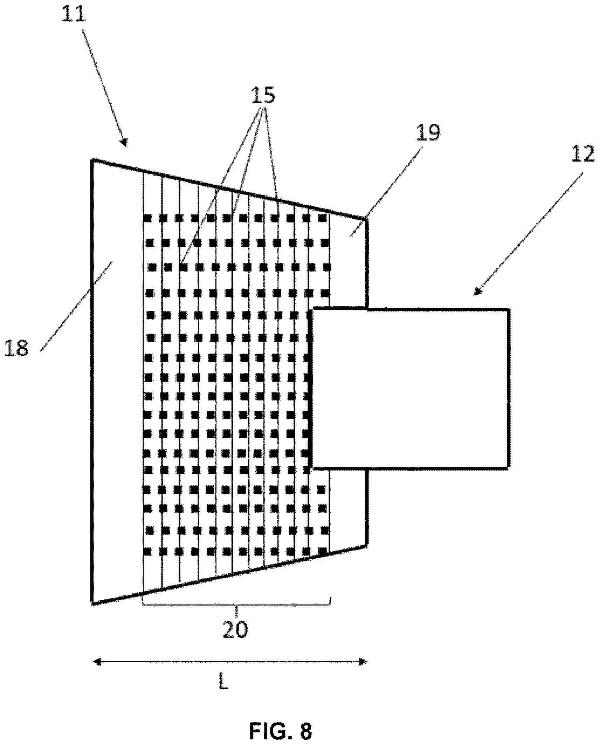

In an alternative embodiment, although less preferred, the discrete bonding elements (15) may be substantially linearly disposed as exemplified in Fig. 8.

In an embodiment, the film (14) is colored with a first color and wherein at least one, preferably both, of the cover layer (13, 13') is colored with a second color, preferably wherein the first color and the second color has a CIELab color space difference (ΔE) of about 16 or less, preferably from 3 to 15, even more preferably from 5 to 12, even more preferably from 6 to 10, wherein the color difference for a first color (L1, a1, b1) and a second color (L2, a2, b2), is calculated according to the following formula: ΔE =√(ΔL2 +Δa2 +Δb2) wherein: ΔL =L1-L2; Δa =a1 -a2; and Δb =b1 -b2. Advantageously, when combined with the wrinkles described herein, this allows to provide a further perception of depth and texture that enhances the overall perception of softness of the material. Color measurements are typically performed using a commercial flat bed scanner capable of 4800 dpi, at 16 bit color depth, such as an Epson Perfection V500 Photo scanner (Epson America, Long Beach, Calif.). Each scan is calibrated against Pantone standards, and measurements made using Adobe Photoshop CS3 Extended Edition (Adobe Systems, Inc, San Jose, Calif.). The sample is measured on the printed side of the substrate. For example, if a laminate consist of a colored nonwoven and a colored film where the film is sandwiched between nonwovens, the nonwoven(s) is removed before the color on the film is measured and then the removed nonwoven is separately measured. Scans are typically calibrated using the Pantone Process Colors standard from the Pantone Formula Guide-Uncoated Papers (Pantone, Carlstadt, N.J.). CIE L*a*b* values are measured for the Pantone standard for each color, i.e., Process Yellow U, Process Magenta U, Process Cyan U, Process Black U, and the White uncoated paper. Tristimulus colors are measured according to ASTM Method E1164-07 (Standard Practice for Obtaining Spectrophotometric Data for Object-Color Evaluation) using a Hunter Labscan XE (HunterLab, Reston, Va.) with HunterLab Universal Software vs. 4.10 with the following settings: Scale CIELAB, 0/45 StdMode, Area View 0.50 in., Port Size 0.70 in., UV filter Nominal During measurement the standard is backed using the white calibration plate provided by HunterLab. To increase the reliability of the measurement, each color should be measured at least in triplicate and averaged. The sample is placed on the scanner with the printed-side toward the sensor. The Pantone standard is also placed on the scanner such that the sample and standard are both captured in the same image. The scan is typically collected at 1200 dpi at 8 bit color depth into Photoshop for objects with a primary dimension of greater than 3 mm, and at 2400 dpi, 8 bit color depth for objects with a primary dimension of less than 3 mm. Within Photoshop, the image is transformed into a Lab, 8 bit image (note in this version of Photoshop, L*a*b* is imprecisely denoted as Lab). Using the "Levels" command, the L channel of the image is adjusted to read within 2 units for each of the yellow, magenta, cyan, black and white colors on the Pantone standard. L*a*b* values are measured using the Color Sampler Tool using an 11 by 11 average sample size.

In an embodiment, the elastomeric film comprises two surfaces and a skin on at least one of the surfaces. Preferably, the skin comprises a polyolefin selected from the group consisting of: metallocene polyethylene, low density polyethylene, high density polyethylene, linear low density polyethylene, very low density polyethylene, a polypropylene homopolymer, a plastic random poly(propylene/olefin) copolymer, syndiotactic polypropylene, metallocene polypropylene, polybutene, an impact copolymer, a polyolefin wax, and mixtures thereof.

The elastomeric film may be pre-activated before attachment to at least one cover layer. Advantageously this allows for increased extension/retraction performance of the laminate in a cost-effective manner.

Preferably, the elastomeric film comprises a polyolefin elastomer, preferably selected from the group consisting of: metallocene polyethylene, low density polyethylene, high density polyethylene, linear low density polyethylene, very low density polyethylene, a polypropylene homopolymer, a plastic random poly(propylene/olefin) copolymer, syndiotactic polypropylene, metallocene polypropylene, polybutene, an impact copolymer, a polyolefin wax, and mixtures thereof.

Typically the elastomeric film is a blown film such as made by use of a blown film extrusion process.

The elastomeric film may have a thickness of from about 15 µm to about 60 µm, preferably from about 20 µm to about 50 µm, preferably from about 30 µm and about 45 µm. The film may comprise an elastomeric polyolefin, and in some embodiments, a polyolefin blown film. Non-limiting examples of useful elastically extensible materials include propylene based homopolymers or co-polymers, or ethylene based homopolymers or co-polymers selected from the group consisting of: an elastic random poly(propylene/olefin) copolymer, an isotactic polypropylene containing stereoerrors, an isotactic/atactic polypropylene block copolymer, an isotactic polypropylene/random poly(propylene/olefin) copolymer block copolymer, a stereoblock elastomeric polypropylene, a syndiotactic polypropylene block poly(ethylene-co-propylene) block syndiotactic polypropylene tri-block copolymer, an isotactic polypropylene block region-irregular polypropylene block isotactic polypropylene tri-block copolymer, a polyethylene random (ethylene/olefin) copolymer block copolymer, a reactor blend polypropylene, a very low density polypropylene, a metallocene polypropylene, metallocene polyethylene, and combinations thereof. Additional non-limiting examples of useful elastically extensible materials include styrene-isoprene-styrene block copolymers, styrene-butadiene-styrene block copolymers, styrene-ethylene-butylene-styrene block copolymers, polyurethanes, ethylene copolymers, polyether block amides, and combinations thereof.

Preferably, the mechanical bonding as referred to in embodiments herein is selected from the group consisting of thermo-sealing, pressure-sealing, ultrasonic bonding, and combinations thereof, preferably ultrasonic bonding.

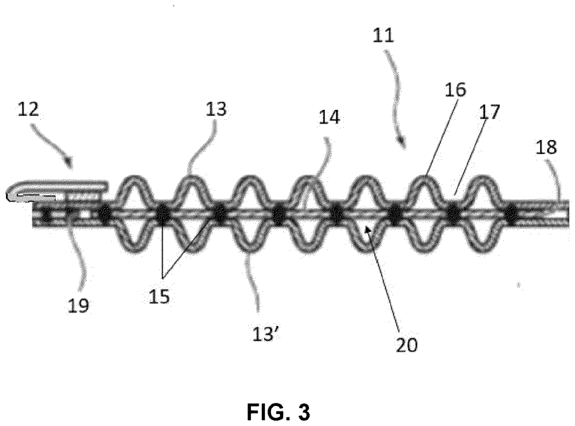

In an embodiment such as illustrated in Fig. 3, the at least one elastically elongatable panel (11) comprises a first and second inelastic region (18, 19) and an elasticized region (20) at least partially disposed between the first and second inelastic regions, and wherein the wrinkles are comprised in said elasticized region (20) and preferably wherein the first and second inelastic regions are free of said wrinkles, preferably wherein the first and second inelastic regions at least partly correspond to the first and second attachment zones respectively.

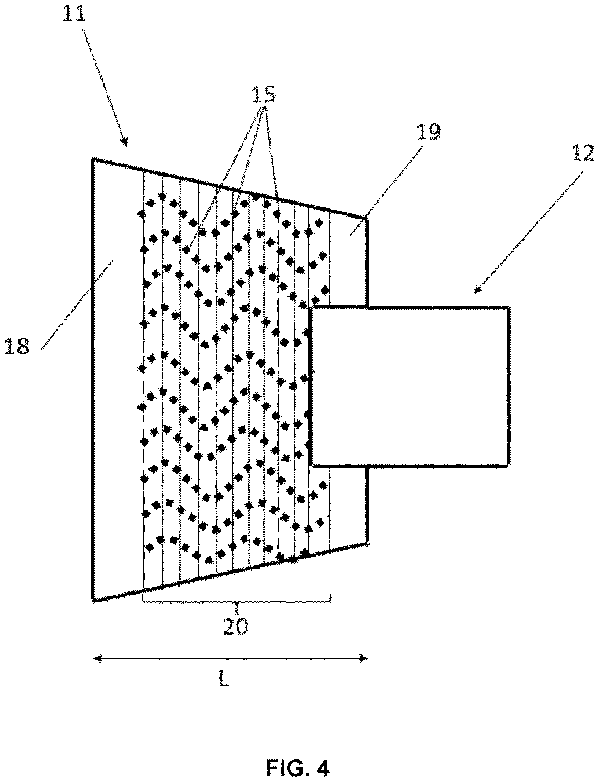

In a highly preferred embodiment as exemplified in Fig. 4, the plurality of discrete bonding elements (15) are disposed in a repeated pattern, preferably located within the elasticized region (20), and wherein said repeated pattern consists of a plurality of sinusoidally oriented discrete bonding elements (15) typically such that a plurality of sinusoidally shaped lines can be traced by connecting said discrete bonding elements (15) with an imaginary line along the panel length (L). Advantageously this allows for a multi-axis bulk-enhancing profile that permits to increase the softness of the laminate without necessarily increasing the basis weight of the cover layers. Moreover, the sinusoidal pattern provides for an increased visual perception of depth that enhances the softness perception of a user. Moreover, such pattern allows to enhance the accordion-like extension and retraction of the laminate.

Preferably, the sinusoidally oriented discrete bonding elements (15) are disposed substantially parallel to each other and each extend along the panel length (L), preferably wherein said sinusoidally oriented discrete bonding elements (15) are spaced apart from each other along an axis substantially perpendicular to said panel length (L).

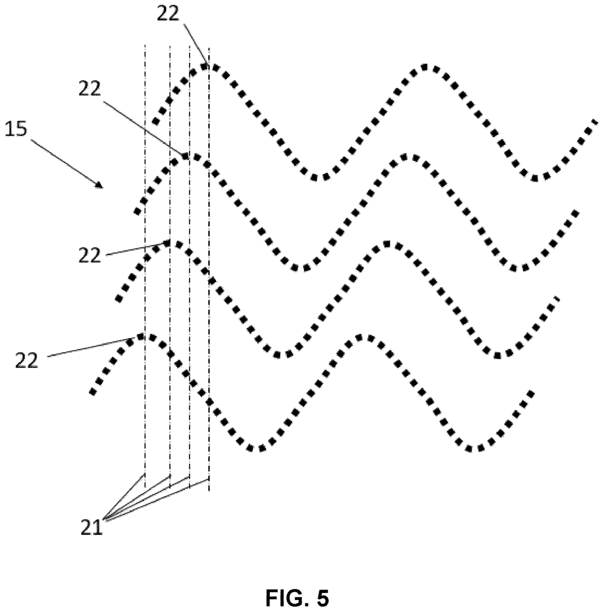

Preferably, as exemplified in Fig. 5, the sinusoidally oriented discrete bonding elements (15) are at least partly offset from each other such that when an imaginary crossing-line (21) is traced substantially perpendicular to the panel length (L) to cross at least one, preferably each, apex (22) of each of at least two, preferably at least three, even more preferably at least four, neighboring sinusoidally oriented discrete bonding elements (15); each said crossing-line is spaced apart from each other along an axis substantially parallel to the panel length (L). Advantageously this arrangement allows for further improved softness and perceived softness by adding a further dimension to the wrinkle formation.

According to the present invention, a first distance between neighboring discrete bonding elements (15) along an axis substantially parallel to the panel length (L) is less than a second distance between neighboring discrete bonding elements (15) along an axis substantially perpendicular to the panel length (L), preferably wherein the ratio of said first and second distances is from 0.10 to 0.80, preferably from 0.15 to 0.70, even more preferably from 0.18 to 0.60, even more preferably from 0.19 to 0.50, even more preferably from 0.20 to 0.45. Advantageously, this arrangement allows for the formation of the desired large amount of wrinkles per unit length whilst at the same time limiting the drawbacks of increased roughness as described above, especially when combined with the above described embodiments comprising a sinusoidal pattern.

In an embodiment the, preferably each, at least one cover layer (13, 13') is a nonwoven preferably having a basis weight of more than 17 g/m², preferably from 18 g/m² to 40 g/m², even more preferably from greater than 22 g/m² to less than 35 g/m², even more preferably from 23 g/m² to 30 g/m², as measured according to ASTM-D3776-9 method C. Preferably, wherein the nonwoven is selected from the group consisting of S, SM, SMS, SMMS, SSMS, and SSMSS, and preferably wherein said nonwoven comprises multi-component fibers typically comprising at least two, preferably at least three of: polyethylene, polypropylene, polyethylene terephthalate, copolyester of polyethylene terephthalate, acrylonitrile butadiene styrene, polylactide, and mixtures thereof. Advantageously this allows for added strength as well as softness of the laminate.

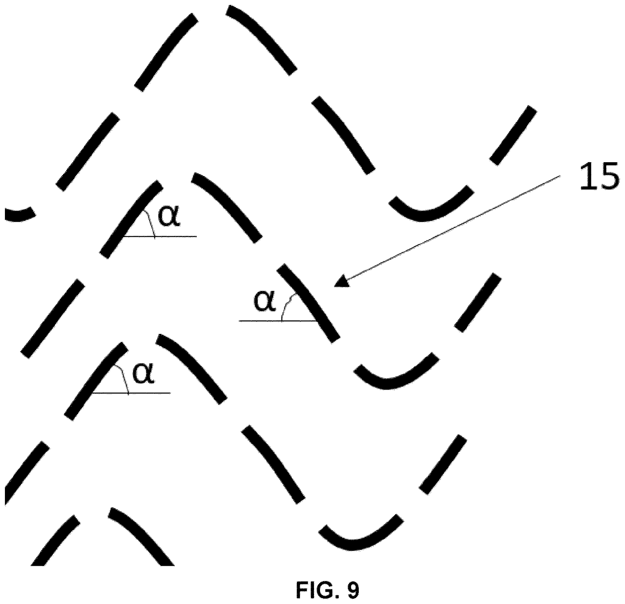

In an embodiment, especially preferable when sinusoidally oriented discrete bonding elements (15) are used in combination with a plurality of elastic strands (14') as described in embodiments herein above, said sinusoidally oriented discrete bonding elements (15) each have a substantially elongate shape preferably having an aspect ratio (i.e. longest dimension divided by shortest dimension) of greater than 1.0, typically from 1.2 to 4.0, preferably from 1.5 to 3.0, and generally wherein each said sinusoidally oriented discrete bonding elements (15) are substantially angled such that an angle (α) is formed between the longest dimension of each said sinusoidally oriented discrete bonding elements (15) and an axis running substantially parallel to the panel length (L) as exemplariy and schematically illustrated in Fig. 9, most preferably wherein said angle (α) is from 5° to 90°, preferably from 10° to 85°, even more preferably from 15° to 80°, even more preferably from 20° to 75°, even more preferably from 25° to 70°. Advantageously this allows for improved anchoring of the elastic strands and reduced risk that some strands do not get anchored and result in too many loose areas especially when producing at high speeds, which may impact both look&feel as well as mechanical stretch performance.

A suitable process that can be used for making elastically elongatable panels herein is described on paragraphs 0063 to 0095 of EP 3 213 728 A1.

The salient modifications required in the taught process include the bonding pattern as described herein and wrinkle formation design.

A suitable activation device that may be employed is described on paragraphs 0041 to 0054 of EP 1 982 823 B1 which is herein incorporated by reference.

### Wrinkle distribution determination

The wrinkle distribution herein is determined by the following method.

For each side panel to be tested, the number of wrinkles is counted over a length of 10mm along the panel length (L) with the laminate in relaxed state (i.e. without applying an extension force between ends thereof, preferably taken at its "unused" state wherein "unused" herein means without the panel ever having been previously stretched since manufacture). The total number of wrinkles is then divided by 10 in order to provide the number of wrinkles per unit length. A total of 4 random locations within the elastic region (20) of the laminate may be measured accordingly and an average is calculated for each sample.

The 10mm length is typically taken from a starting position that encompasses at least one full wrinkle (i.e. a complete wrinkle comprising a peak positioned between two consecutive troughs) and by doing so if at the opposite extremity of the 10mm length only a partial wrinkle (i.e. not a complete wrinkle comprising a peak positioned between two consecutive troughs, e.g. the 10mm end at a position corresponding to the peak of a wrinkle) is formed, this is not counted as a wrinkle within the present method.

The above procedure is typically repeated for at least 4 samples of side panels and an average is calculated to provide the wrinkle distribution in wrinkles/mm as referred to herein.

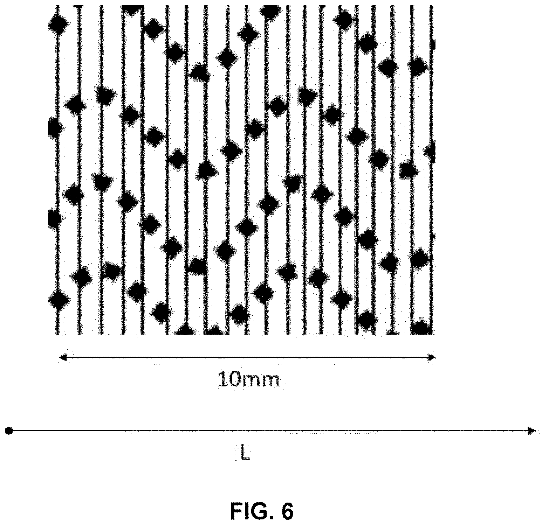

As an example, the schematic of Fig. 6 illustrates 20 wrinkles over a length of 10mm along the panel length (L) with the laminate in relaxed state. If the same is observed at the 4 locations within the same sample and after examining a total of at least 4 samples, the wrinkle distribution in this example is 2 wrinkles/mm. Of course, it is understood herein that in case of variations/differences in number of wrinkles observed at the respective locations and/or samples, the wrinkle distribution is taken as the average as explained above.

It is supposed that the present invention is not restricted to any form of realization described previously and that some modifications can be added to the presented embodiments without reappraisal of the appended claims.

## Claims

1. An absorbent article (1), the absorbent article selected from a diaper or pant, comprising:
a chassis comprising a liquid permeable topsheet (2), a liquid impermeable backsheet (3), and an absorbent core (4) comprising absorbent material selected from the group consisting of cellulose fibers and superabsorbent polymers, said absorbent core positioned between said topsheet (2) and backsheet (3), and comprising a longitudinal axis (y) extending along a length of said chassis and a transverse axis being substantially perpendicular to the longitudinal axis and extending along a width of said chassis, wherein said length extends along the longest dimension of said chassis; and
at least one elastically elongatable panel (11) joined to the chassis, wherein the elastically elongatable panel comprises:
a. at least one cover layer (13, 13'); and
b. an elastic material selected from the group consisting of an elastomeric film (14), and a plurality of elastic strands (14'); the elastic material attached to the cover layer (13, 13'),
wherein said least one cover layer (13, 13') and elastic material are joined by mechanical bonding, at a plurality of discrete bonding elements (15) and wherein each said panel (11) comprises a plurality of wrinkles having peaks (16) and troughs (17) formed on at least one of said cover layer (13, 13'),
wherein said panel (11) comprises an average of no more than two, preferably no more than one, wrinkles between two consecutive discrete bonding elements (15) along a panel length (L) extending substantially parallel to the transverse axis, and **characterised in that** said panel (11) comprises a wrinkle distribution of at least 1.1 wrinkles/mm, according to the method herein; and wherein a first distance between neighbouring discrete bonding elements (15) along an axis substantially parallel to the panel length (L) is less than a second distance between neighbouring discrete bonding elements (15) along an axis substantially perpendicular to the panel length (L).

2. An absorbent article (1) according to Claim 1 wherein the plurality of discrete bonding elements (15) are sinusoidally disposed are aligned such that when one or more imaginary lines are traced between bonding elements substantially sinusoidal waves are formed along the panel length (L), and wherein each of the plurality of elastic strands (14') crosses said imaginary line at a plurality of intersections, preferably at least 4 intersections, even more preferably at least 5 intersections, even more preferably from 6 to 30 intersections, even more preferably from 7 to 25 intersections, even more preferably from 8 to 20 intersections, even more preferably from 9 to 15 intersections

3. An absorbent article (1) according to any of the preceding Claims wherein the elastomeric film (14), or elastomeric strands (14'), comprises two surfaces and a skin on at least one of the surfaces.

4. An absorbent article (1) according to any of the preceding Claims comprising at least two, elastically elongatable panel (11) joined to the chassis.

5. An absorbent article (1) according to any of the preceding Claims wherein the elastomeric film (14), or elastic strands (14'), comprises a polyolefin elastomer, preferably selected from the group consisting of: metallocene polyethylene, low density polyethylene, high density polyethylene, linear low density polyethylene, very low density polyethylene, a polypropylene homopolymer, a plastic random poly(propylene/olefin) copolymer, syndiotactic polypropylene, metallocene polypropylene, polybutene, an impact copolymer, a polyolefin wax, and mixtures thereof.

6. An absorbent article (1) according to any of the preceding Claims wherein the at least one elastically elongatable panel (11) is joined to the chassis by mechanical bonding or adhesive at a first attachment zone of said panel, and preferably wherein said panel (11) is joined, preferably by mechanical bonding or adhesive, to a fastening member (12) at a second attachment zone of said panel oppositely disposed from said first attachment zone at an opposite end of said panel.

7. An absorbent article (1) according to any of the preceding Claims wherein the mechanical bonding is selected from the group consisting of thermo-sealing, pressure-sealing, ultrasonic bonding, and combinations thereof, preferably ultrasonic bonding.

8. An absorbent article (1) according to any of the preceding Claims wherein the elastomeric film, or elastic strands, is pre-activated.

9. An absorbent article (1) according to any of the preceding Claims wherein the at least one elastically elongatable panel (11) comprises a first and second inelastic region (18, 19) and an elasticized region (20) at least partially disposed between the first and second inelastic regions, and wherein the wrinkles are comprised in said elasticized region (20) and preferably wherein the first and second inelastic regions are free of said wrinkles, preferably wherein the first and second inelastic regions at least partly correspond to the first and second attachment zones respectively.

10. An absorbent article (1) according to any of the preceding Claims wherein the plurality of discrete bonding elements (15) are disposed in a repeated pattern, preferably located within the elasticized region (20), and wherein said repeated pattern consists of a plurality of sinusoidally oriented discrete bonding elements (15) typically such that a plurality of sinusoidally shaped lines can be traced by connecting said discrete bonding elements (15) with an imaginary line along the panel length (L).

11. An absorbent article (1) according to Claim 10 wherein the sinusoidally oriented discrete bonding elements (15) are disposed substantially parallel to each other and each extend along the panel length (L), preferably wherein said sinusoidally oriented discrete bonding elements (15) are spaced apart from each other along an axis substantially perpendicular to said panel length (L).

12. An absorbent article (1) according to Claims 10 to 11 wherein the sinusoidally oriented discrete bonding elements (15) are at least partly offset from each other such that when an imaginary crossing-line (21) is traced substantially perpendicular to the panel length (L) to cross at least one, preferably each, apex (22) of each of at least two, preferably at least three, even more preferably at least four, neighboring sinusoidally oriented discrete bonding elements (15); each said crossing-line (21) is spaced apart from each other along an axis substantially parallel to the panel length (L).

13. An absorbent article (1) according to any of the preceding Claims, wherein the ratio of said first and second distances is from 0.10 to 0.80, preferably from 0.15 to 0.70, even more preferably from 0.18 to 0.60, even more preferably from 0.19 to 0.50, even more preferably from 0.20 to 0.45.

14. An absorbent article (1) according to any of the preceding Claims wherein the, preferably each, at least one cover layer (13, 13') is a nonwoven preferably having a basis weight of more than 17 g/m², preferably from 18 g/m² to 40 g/m², even more preferably from greater than 22 g/m² to less than 35 g/m², even more preferably from 23 g/m² to 30 g/m², as measured according to ASTM-D3776-9 method C.

15. An absorbent article (1) according to Claim 14 wherein the nonwoven is selected from the group consisting of S, SM, SMS, SMMS, SSMS, and SSMSS, and preferably wherein said nonwoven comprises multi-component fibers typically comprising at least two, preferably at least three of: polyethylene, polypropylene, polyethylene terephthalate, copolyester of polyethylene terephthalate, acrylonitrile butadiene styrene, polylactide, and mixtures thereof.

## Patentansprüche

1. Absorptionsartikel (1), wobei der Absorptionsartikel aus einer Windel oder einer Hose ausgewählt ist, umfassend:
ein Chassis, umfassend eine flüssigkeitsdurchlässige Oberschicht (2), eine flüssigkeitsundurchlässige Unterschicht (3) und einen Absorptionskern (4), umfassend Absorptionsmaterial, das ausgewählt ist aus der Gruppe bestehend aus Zellulosefasern und Superabsorptionspolymeren, wobei der Absorptionskern zwischen der Oberschicht (2) und der Unterschicht (3) positioniert ist und umfassend eine Längsachse (y), die sich entlang einer Länge des Chassis erstreckt, und eine Querachse, die im Wesentlichen senkrecht zu der Längsachse ist und sich entlang einer Breite des Chassis erstreckt, wobei sich die Länge entlang der längsten Abmessung des Chassis erstreckt; und
mindestens ein elastisch verlängerbares Panel (11), das mit dem Chassis zusammengefügt ist, wobei das elastisch verlängerbare Panel umfasst:
a. mindestens eine Deckschicht (13, 13'); und
b. ein elastisches Material, ausgewählt aus der Gruppe bestehend aus einer Elastomerfolie (14) und einer Vielzahl von elastischen Strängen (14'); wobei das elastische Material an der Deckschicht (13, 13') angebracht ist,
wobei die mindestens eine Deckschicht (13, 13') und das elastische Material durch mechanisches Binden an einer Vielzahl von diskreten Bindungselementen (15) zusammengefügt sind, und wobei jedes Panel (11) eine Vielzahl von Falten, die Spitzen (16) und Vertiefungen (17) aufweisen, die auf mindestens einer der Deckschichten (13, 13') ausgebildet sind, umfasst,
wobei das Panel (11) im Durchschnitt nicht mehr als zwei, vorzugsweise nicht mehr als eine Falte zwischen zwei aufeinanderfolgenden diskreten Bindungselementen (15) entlang einer Panellänge (L) umfasst, die sich im Wesentlichen parallel zu der Querachse erstreckt, und
**dadurch gekennzeichnet, dass** das Panel (11) eine Faltenverteilung von mindestens 1,1 Falten/mm; gemäß dem hier beschriebenen Verfahren, umfasst;
und wobei ein erster Abstand zwischen benachbarten diskreten Bindungselementen (15) entlang einer Achse, die im Wesentlichen parallel zu der Panellänge (L) verläuft, kleiner als ein zweiter Abstand zwischen benachbarten diskreten Bindungselementen (15) entlang einer Achse, die im Wesentlichen senkrecht zu der Panellänge (L) verläuft, ist.

2. Absorptionsartikel (1) nach Anspruch 1, wobei die Vielzahl von diskreten Bindungselementen (15) sinusförmig angeordnet sind, derart ausgerichtet sind, dass wenn eine oder mehrere imaginäre Linien zwischen den Bindungselementen nachgezeichnet werden, im Wesentlichen sinusförmige Wellen entlang der Panellänge (L) ausgebildet werden, und wobei jeder der Vielzahl von elastischen Strängen (14') die imaginäre Linie an einer Vielzahl von Schnittpunkten kreuzt, vorzugsweise an mindestens 4 Schnittpunkten, noch mehr bevorzugt an mindestens 5 Schnittpunkten, noch mehr bevorzugt von 6 bis 30 Schnittpunkten, noch mehr bevorzugt von 7 bis 25 Schnittpunkten, noch mehr bevorzugt von 8 bis 20 Schnittpunkten, noch mehr bevorzugt von 9 bis 15 Schnittpunkten.

3. Absorptionsartikel (1) nach einem der vorstehenden Ansprüche, wobei die Elastomerfolie (14) oder die Elastomerstränge (14') zwei Oberflächen und eine Haut auf mindestens einer der Oberflächen umfassen.

4. Absorptionsartikel (1) nach einem der vorstehenden Ansprüche, umfassend mindestens zwei elastisch verlängerbare Panele (11), die mit dem Chassis zusammengefügt sind.

5. Absorptionsartikel (1) nach einem der vorstehenden Ansprüche, wobei die Elastomerfolie (14) oder die elastischen Stränge (14') ein Polyolefin-Elastomer umfassen, das vorzugsweise aus der Gruppe ausgewählt ist, bestehend aus: Metallocen-Polyethylen, Polyethylen niedriger Dichte, Polyethylen hoher Dichte, linearem Polyethylen niedriger Dichte, Polyethylen sehr niedriger Dichte, einem Polypropylen-Homopolymer, einem plastischen statistischen Poly(propylen/olefin)-Copolymer, syndiotaktischem Polypropylen, Metallocen-Polypropylen, Polybuten, einem schlagfesten Copolymer, einem Polyolefinwachs und Mischungen davon.

6. Absorptionsartikel (1) nach einem der vorstehenden Ansprüche, wobei das mindestens eine elastisch verlängerbare Panel (11) mit dem Chassis durch mechanisches Binden oder Klebstoff an einer ersten Anbringungszone des Panels zusammengefügt ist, und vorzugsweise wobei das Panel (11) vorzugsweise durch mechanisches Binden oder Klebstoff mit einem Befestigungselement (12) an einer zweiten Anbringungszone des Panels, die gegenüber von der Anbringungszone an einem gegenüberliegenden Ende des Panels angeordnet ist, zusammengefügt ist.

7. Absorptionsartikel (1) nach einem der vorstehenden Ansprüche, wobei das mechanische Binden aus der Gruppe ausgewählt ist, bestehend aus Thermoversiegelung, Druckversiegelung, Ultraschallbindung und Kombinationen davon, vorzugsweise Ultraschallbindung.

8. Absorptionsartikel (1) nach einem der vorstehenden Ansprüche, wobei die Elastomerfolie oder die elastischen Stränge voraktiviert sind.

9. Absorptionsartikel (1) nach einem der vorstehenden Ansprüche, wobei die mindestens ein elastisch verlängerbares Panel (11) eine erste und eine zweite unelastische Region (18, 19) und eine elastifizierte Region (20), die mindestens teilweise zwischen der ersten und der zweiten unelastischen Region angeordnet ist, umfasst, und wobei die Falten in der elastifizierten Region (20) enthalten sind, und vorzugsweise wobei die erste und die zweite unelastische Region frei von diesen Falten sind, vorzugsweise wobei die erste und die zweite unelastische Region mindestens teilweise der ersten bzw. der zweiten Anbringungszone entsprechen.

10. Absorptionsartikel (1) nach einem der vorstehenden Ansprüche, wobei die Vielzahl von diskreten Bindungselementen (15) in einem Wiederholungsmuster angeordnet sind, das sich vorzugsweise innerhalb der elastifizierten Region (20) befindet, und wobei das Wiederholungsmuster aus einer Vielzahl von sinusförmig ausgerichteten diskreten Bindungselementen (15) besteht, üblicherweise derart, dass eine Vielzahl von sinusförmigen Linien nachgezeichnet werden können, durch Verbinden der diskreten Bindungselemente (15) mit einer imaginären Linie entlang der Panellänge (L).

11. Absorptionsartikel (1) nach Anspruch 10, wobei die sinusförmig ausgerichteten diskreten Bindungselemente (15) im Wesentlichen parallel zueinander angeordnet sind und sich jeweils entlang der Panellänge (L) erstrecken, vorzugsweise wobei die sinusförmig ausgerichteten diskreten Bindungselemente (15) entlang einer Achse, die im Wesentlichen senkrecht zu der Panellänge (L) verläuft, voneinander beabstandet sind.

12. Absorptionsartikel (1) nach den Ansprüchen 10 bis 11, wobei die sinusförmig ausgerichteten diskreten Bindungselemente (15) mindestens teilweise derart voneinander versetzt sind, dass, wenn eine imaginäre Kreuzungslinie (21) im Wesentlichen senkrecht zu der Panellänge (L) nachgezeichnet wird, diese mindestens einen, vorzugsweise jeden Scheitelpunkt (22) von jedem von mindestens zwei, vorzugsweise mindestens drei, noch mehr bevorzugt mindestens vier benachbarte sinusförmig ausgerichtete diskrete Bindungselemente (15) kreuzen soll; wobei jede Kreuzungslinie (21) entlang einer Achse, die im Wesentlichen parallel zu der Panellänge (L) verläuft, voneinander beabstandet sind.

13. Absorptionsartikel (1) nach einem der vorstehenden Ansprüche, wobei das Verhältnis des ersten und des zweiten Abstands 0,10 bis 0,80, vorzugsweise von 0,15 bis 0,70, noch mehr bevorzugt von 0,18 bis 0,60, noch mehr bevorzugt von 0,19 bis 0,50, noch mehr bevorzugt von 0,20 bis 0,45 beträgt.

14. Absorptionsartikel (1) nach einem der vorstehenden Ansprüche, wobei die, vorzugsweise jede, mindestens eine Deckschicht (13, 13') ein Vlies ist, das vorzugsweise ein Flächengewicht von mehr als 17 g/m², vorzugsweise von 18 g/m² bis 40 g/m², noch mehr bevorzugt von mehr als 22 g/m² bis weniger als 35 g/m², noch mehr bevorzugt von 23 g/m² bis 30 g/m², gemessen nach ASTM-D3776-9 Methode C, aufweist.

15. Absorptionsartikel (1) nach Anspruch 14, wobei das Vlies aus der Gruppe ausgewählt ist, bestehend aus S, SM, SMS, SMMS, SSMS und SSMSS, und vorzugsweise wobei das Vlies Mehrkomponentenfasern umfasst, üblicherweise umfassend mindestens zwei, vorzugsweise mindestens drei von: Polyethylen, Polypropylen, Polyethylenterephthalat, Copolyester von Polyethylenterephthalat, Acrylnitril-Butadien-Styrol, Polylactid und Mischungen davon.

## Revendications

1. Article absorbant (1), l'article absorbant choisi dans une couche ou une culotte, comprenant :
un châssis comprenant une feuille supérieure perméable aux liquides (2), une feuille de fond imperméable aux liquides (3) et une âme absorbante (4) comprenant un matériau absorbant choisi dans le groupe constitué de fibres de cellulose et de polymères superabsorbants, ladite âme absorbante placée entre ladite feuille supérieure (2) et ladite feuille de fond (3), et comprenant un axe longitudinal (y) s'étendant le long d'une longueur dudit châssis et un axe transversal étant sensiblement perpendiculaire à l'axe longitudinal et s'étendant le long d'une largeur dudit châssis, dans lequel ladite longueur s'étend le long de la dimension la plus longue dudit châssis ; et
au moins un panneau élastiquement extensible (11) relié au châssis, dans lequel le panneau élastiquement extensible comprend :
a. au moins une couche de couverture (13, 13') ; et
b. un matériau élastique choisi dans le groupe constitué d'un film élastomère (14) et d'une pluralité de fils élastiques (14') ; le matériau élastique attaché à la couche de couverture (13, 13'),
dans lequel ladite au moins une couche de couverture (13, 13') et le matériau élastique sont reliés par liaison mécanique, au niveau d'une pluralité d'éléments de liaison distincts (15) et dans lequel chaque dit panneau (11) comprend une pluralité de plis présentant des pics (16) et des creux (17) formés sur au moins l'une de ladite couche de couverture (13, 13'),
dans lequel ledit panneau (11) comprend une moyenne de pas plus de deux, de préférence pas plus d'un, plis entre deux éléments de liaison distincts (15) consécutifs le long d'une longueur de panneau (L) s'étendant sensiblement parallèlement à l'axe transversal, et **caractérisé en ce que** ledit panneau (11) comprend une distribution de plis d'au moins 1,1 pli/mm, selon le présent procédé ; et dans lequel une première distance entre des éléments de liaison distincts (15) voisins le long d'un axe sensiblement parallèle à la longueur de panneau (L) est inférieure à une seconde distance entre des éléments de liaison distincts (15) voisins le long d'un axe sensiblement perpendiculaire à la longueur de panneau (L).

2. Article absorbant (1) selon la revendication 1, dans lequel la pluralité d'éléments de liaison distincts (15) sont disposés de manière sinusoïdale et alignés de sorte que lorsqu'une ou plusieurs lignes imaginaires sont tracées entre les éléments de liaison, des ondes sensiblement sinusoïdales sont formées le long de la longueur de panneau (L), et dans lequel chacun parmi la pluralité de fils élastiques (14') traverse ladite ligne imaginaire au niveau d'une pluralité d'intersections, de préférence au moins 4 intersections, encore plus préférablement au moins 5 intersections, encore plus préférablement de 6 à 30 intersections, encore plus préférablement de 7 à 25 intersections, encore plus préférablement de 8 à 20 intersections, encore plus préférablement de 9 à 15 intersections

3. Article absorbant (1) selon l'une quelconque des revendications précédentes, dans lequel le film élastomère (14), ou les fils élastomères (14'), comprend deux surfaces et une peau sur au moins l'une des surfaces.

4. Article absorbant (1) selon l'une quelconque des revendications précédentes, comprenant au moins deux panneaux élastiquement extensibles (11) reliés au châssis.

5. Article absorbant (1) selon l'une quelconque des revendications précédentes, dans lequel le film élastomère (14), ou les fils élastiques (14'), comprend un élastomère de polyoléfine, de préférence choisi dans le groupe constitué de : polyéthylène métallocène, polyéthylène basse densité, polyéthylène haute densité, polyéthylène basse densité linéaire, polyéthylène très basse densité, homopolymère de polypropylène, copolymère plastique aléatoire de poly(propylène/oléfine), polypropylène syndiotactique, polypropylène métallocène, polybutène, copolymère résistant aux chocs, cire de polyoléfine, et mélanges de ceux-ci.

6. Article absorbant (1) selon l'une quelconque des revendications précédentes, dans lequel l'au moins un panneau élastiquement extensible (11) est relié au châssis par liaison mécanique ou adhésif au niveau d'une première zone d'attache dudit panneau, et de préférence dans lequel ledit panneau (11) est relié, de préférence par liaison mécanique ou adhésif, à un élément de fixation (12) au niveau d'une seconde zone d'attache dudit panneau disposée à l'opposé de ladite première zone d'attache au niveau d'une extrémité opposée dudit panneau.

7. Article absorbant (1) selon l'une quelconque des revendications précédentes, dans lequel la liaison mécanique est choisie dans le groupe constitué de thermoscellage, scellage par pression, liaison ultrasonore et combinaisons de ceux-ci, de préférence liaison ultrasonore.

8. Article absorbant (1) selon l'une quelconque des revendications précédentes, dans lequel le film élastomère, ou les fils élastiques, est préactivé.

9. Article absorbant (1) selon l'une quelconque des revendications précédentes, dans lequel l'au moins un panneau élastiquement extensible (11) comprend une première et une seconde région inélastique (18, 19) et une région élastiquée (20) au moins partiellement disposée entre les première et seconde régions inélastiques, et dans lequel les plis sont compris dans ladite région élastiquée (20) et de préférence dans lequel les première et seconde régions inélastiques sont exemptes desdits plis, de préférence dans lequel les première et seconde régions inélastiques correspondent au moins partiellement aux première et seconde zones d'attache respectivement.

10. Article absorbant (1) selon l'une quelconque des revendications précédentes, dans lequel la pluralité d'éléments de liaison distincts (15) sont disposés selon un motif répété, de préférence situé dans la région élastiquée (20), et dans lequel ledit motif répété est constitué d'une pluralité d'éléments de liaison distincts (15) orientés de manière sinusoïdale, de manière générale de sorte qu'une pluralité de lignes formées de manière sinusoïdale peuvent être tracées en reliant lesdits éléments de liaison distincts (15) à une ligne imaginaire le long de la longueur de panneau (L).

11. Article absorbant (1) selon la revendication 10, dans lequel les éléments de liaison distincts (15) orientés de manière sinusoïdale sont disposés sensiblement parallèlement les uns aux autres et s'étendent chacun le long de la longueur de panneau (L), de préférence dans lequel lesdits éléments de liaison distincts (15) orientés de manière sinusoïdale sont espacés les uns des autres le long d'un axe sensiblement perpendiculaire à ladite longueur de panneau (L).

12. Article absorbant (1) selon les revendications 10 à 11, dans lequel les éléments de liaison distincts (15) orientés de manière sinusoïdale sont au moins partiellement décalés les uns par rapport aux autres de sorte que lorsqu'une ligne de croisement imaginaire (21) est tracée sensiblement perpendiculairement à la longueur de panneau (L) pour traverser au moins un, de préférence chaque, apex (22) de chacun parmi au moins deux, de préférence au moins trois, encore plus préférablement au moins quatre, éléments de liaison distincts (15) orientés de manière sinusoïdale voisins ; chaque dite ligne de croisement (21) est espacée par rapport à l'autre le long d'un axe sensiblement parallèle à la longueur de panneau (L).

13. Article absorbant (1) selon l'une quelconque des revendications précédentes, dans lequel le rapport entre lesdites première et seconde distances est compris entre 0,10 et 0,80, de préférence entre 0,15 et 0,70, encore plus préférablement entre 0,18 et 0,60, encore plus préférablement entre 0,19 et 0,50, encore plus préférablement entre 0,20 et 0,45.

14. Article absorbant (1) selon l'une quelconque des revendications précédentes, dans lequel la, de préférence chaque, au moins une couche de couverture (13, 13') est un non-tissé ayant de préférence un poids de base supérieur à 17 g/m², de préférence compris entre 18 g/m² et 40 g/m², encore plus préférablement entre plus de 22 g/m² et moins de 35 g/m², encore plus préférablement entre 23 g/m² et 30 g/m², tel que mesuré selon la méthode C de la norme ASTM-D3776-9.

15. Article absorbant (1) selon la revendication 14, dans lequel le non-tissé est choisi dans le groupe constitué de S, SM, SMS, SMMS, SSMS et SSMSS, et de préférence dans lequel ledit non-tissé comprend des fibres à composants multiples comprenant de manière générale au moins deux, de préférence au moins trois parmi : polyéthylène, polypropylène, polytéréphtalate d'éthylène, copolyester de polytéréphtalate d'éthylène, acrylonitrile butadiène styrène, polylactide, et mélanges de ceux-ci.
